# EUROPEAN PATENT APPLICATION

(11) **EP 3 949 862 A1**
(43) Date of publication of application: **09.02.2022**
(21) Application number: 19922862.8
(22) Date of filing: 23.10.2019
(51) Int. Cl.: A61B 6/02, A61B 6/00

(54) **IMAGE PROCESSING DEVICE, METHOD, AND PROGRAM**

(30) Priority: 29.03.2019 JP 2019067746
(71) Applicant: FUJIFILM Corporation, Tokyo 106-8620 (JP)
(72) Inventor: KOIKE, Takafumi, Ashigarakami-gun, Kanagawa 258-8538 (JP)
(74) Representative: Kudlek, Franz Thomas
(86) International application number: PCT/JP2019/041480
(87) International publication number: WO 2020/202612

(57) **Abstract**

A structure extraction unit extracts a specific structure from each of a plurality of tomographic images representing a plurality of tomographic planes of a subject. A composition unit generates a composite two-dimensional image from the plurality of tomographic images by setting a weight of a pixel of the specific structure in each of the plurality of tomographic images to be larger than a weight of a pixel other than the pixel of the specific structure.

## Description

### BACKGROUND

### Technical Field

The present disclosure relates to an image processing apparatus, an image processing method, and an image processing program.

### Related Art

As a method for imaging a radiographic image, there is known so-called tomosynthesis imaging of sequentially irradiating a subject with radiation at each of a plurality of irradiation positions with different irradiation angles and imaging a plurality of projection images by a radiation detector for each irradiation position. Further, there is known a technique of generating tomographic images by performing reconfiguration processing of the plurality of projection images acquired by tomosynthesis imaging.

In addition, there is known a technique of generating, using a plurality of tomographic images with different distances (positions in a height direction) from a detection surface of a radiation detector toward a radiation source, a pseudo two-dimensional image (hereinafter, referred to as a composite two-dimensional image) corresponding to a two-dimensional image (hereinafter, referred to as a simple two-dimensional image) obtained by irradiating a subject with radiation in a state where irradiation positions of the radiation source are fixed and imaging the subject (refer to JP5952251B).

On the other hand, in a medical field, there is known a computer aided diagnosis (CAD, hereinafter referred to as CAD) system that automatically detects an abnormal shadow such as a lesion in an image and highlights the detected abnormal shadow. For example, an important structure in diagnosis, such as a spicula and a tumor, is extracted from a breast image by using CAD. In addition, in a case of generating a composite two-dimensional image from a plurality of tomographic images acquired by tomosynthesis imaging of a breast, there is proposed a method of extracting interest regions including a lesion by using CAD and composing the extracted interest regions on the composite two-dimensional image (refer to US8983156B). Further, there is proposed a method of specifying an image having best characteristics by comparing characteristics of common regions in a plurality of images including tomographic images and generating a composite two-dimensional image by composing common regions of the specified image (refer to US2014/0327702A). Further, there is proposed a method of detecting an edge from a tomographic image, generating a weight region by expanding the detected edge, and generating a composite two-dimensional image in consideration of a weight of the weight region (refer to US10140715B).

### SUMMARY OF THE INVENTION

On the other hand, among structures included in a breast, a linear structure such as a spicula is an important structure for diagnosis (hereinafter, referred to as a specific structure). Thus, a linear structure needs to be included in a composite two-dimensional image so as to be clear. However, in the methods described in US8983156B and US2014/0327702A, in a case of composing the extracted interest regions or the extracted structures on a composite two-dimensional image, signal values of the linear structure are averaged. For this reason, the linear structure may be hidden in other regions on the composite two-dimensional image, and as a result, it may be difficult to find the linear structure. Further, in the method described in US10140715B, in the tomographic images which are above and below a linear structure such as a spicula, edges such as normal mammary glands included in the tomographic images are detected. On the other hand, in the composite two-dimensional image, the edges such as mammary glands overlap with a spicula, and as a result, it is difficult to find the spicula. Further, in the method described in US10140715B, the detected edges such as mammary glands may overlap with each other, and as a result, a structure that looks like a spicula may appear in the composite two-dimensional image.

### SUMMARY OF THE INVENTION

The present disclosure has been made in view of the above circumstances, and an object of the present disclosure is to make it possible to easily find a specific structure included in a tomographic image in a composite two-dimensional image.

According to an aspect of the present disclosure, there is provided an image processing apparatus including: a structure extraction unit that extracts a specific structure from each of a plurality of tomographic images representing a plurality of tomographic planes of a subject; and a composition unit that generates a composite two-dimensional image from the plurality of tomographic images by setting a weight of a pixel of the specific structure in each of the plurality of tomographic images to be larger than a weight of a pixel other than the pixel of the specific structure.

In the image processing apparatus according to the aspect of the present disclosure, the composition unit may set, in pixels other than the pixel of the specific structure in each of the plurality of tomographic images, a weight of a corresponding pixel corresponding to the specific structure included in other tomographic images to be smaller than a weight of a pixel other than the corresponding pixel.

In this case, the composition unit may set the weight of the corresponding pixel to 0.

In the image processing apparatus according to the aspect of the present disclosure, the composition unit may set the weight of the specific structure in each of the plurality of tomographic images to 1.

In this case, the composition unit may normalize a pixel value of the specific structure on the composite two-dimensional image based on a maximum value of a pixel value that is allowed for the composite two-dimensional image.

In the image processing apparatus according to the aspect of the present disclosure, the composition unit may generate the composite two-dimensional image by applying the weight to a pixel value of a corresponding pixel of the plurality of tomographic images and adding the weighted pixel value.

The image processing apparatus according to the aspect of the present disclosure further includes: an image acquisition unit that acquires a plurality of projection images corresponding to each of a plurality of radiation source positions, the plurality of projection images being generated by causing an imaging apparatus to perform tomosynthesis imaging of relatively moving a radiation source with respect to a detection surface of a detection unit and irradiating the subject with radiation at the plurality of radiation source positions according to movement of the radiation source; and a reconfiguration unit that generates the plurality of tomographic images by reconfiguring the plurality of projection images.

The image processing apparatus according to the aspect of the present disclosure may further include a position deviation correction unit that corrects a position deviation between the plurality of projection images. The reconfiguration unit may generate the plurality of tomographic images by reconfiguring the plurality of projection images obtained by correcting the position deviation.

In the image processing apparatus according to the aspect of the present disclosure, the composition unit may perform smoothing processing on a boundary of the specific structure in the composite two-dimensional image.

The "smoothing processing" means processing of matching a pixel value near a boundary of the specific structure with a pixel value of a background in contact with the boundary of the specific structure by smoothly changing the pixel value near the boundary of the specific structure.

In the image processing apparatus according to the aspect of the present disclosure, the composition unit may generate the composite two-dimensional image by, for pixels other than the pixel of the specific structure in each of the plurality of tomographic images and a corresponding pixel corresponding to the pixel of the specific structure included in other tomographic images, lowering a weight of a noise pixel which is greatly influenced by a noise than the pixel of the specific structure, or by excluding the noise pixel.

In the image processing apparatus according to the aspect of the present disclosure, the subject may be a breast, and the specific structure may be a spicula and a tumor.

According to another aspect of the present disclosure, there is provided an image processing method including: extracting a specific structure from each of a plurality of tomographic images representing a plurality of tomographic planes of a subject; and generating a composite two-dimensional image from the plurality of tomographic images by setting a weight of a pixel of the specific structure in each of the plurality of tomographic images to be larger than a weight of a pixel other than the pixel of the specific structure.

A program causing a computer to execute the image processing method according to the aspect of the present disclosure may be provided.

According to still another aspect of the present disclosure, there is provided an image processing apparatus including: a memory that stores a command to be executed by a computer; and a processor configured to execute the stored command. The processor is configured to execute processing of extracting a specific structure from each of a plurality of tomographic images representing a plurality of tomographic planes of a subject, and generating a composite two-dimensional image from the plurality of tomographic images by setting a weight of a pixel of the specific structure in each of the plurality of tomographic images to be larger than a weight of a pixel other than the pixel of the specific structure.

According to the present disclosure, it is possible to easily find a specific structure in a composite two-dimensional image.

### BRIEF DESCRIPTION OF THE DRAWINGS

Fig. 1 is a schematic configuration diagram of a radiography apparatus to which an image processing apparatus according to an embodiment of the present disclosure is applied.
Fig. 2 is a diagram illustrating the radiography apparatus as viewed from a direction of an arrow A in Fig. 1.
Fig. 3 is a diagram illustrating a schematic configuration of the image processing apparatus realized by installing, in a computer, an image processing program according to the present embodiment.
Fig. 4 is a diagram for explaining acquisition of projection images.
Fig. 5 is a diagram for explaining generation of tomographic images.
Fig. 6 is a diagram for explaining extraction of a specific structure from the tomographic images.
Fig. 7 is a diagram for explaining generation of a composite two-dimensional image.
Fig. 8 is a diagram for explaining setting of weights.
Fig. 9 is a diagram for explaining setting of weights.
Fig. 10 is a diagram illustrating a profile of signal values of a corresponding region in a composite two-dimensional image.
Fig. 11 is a diagram for explaining generation of a composite two-dimensional image by set weights.
Fig. 12 is a flowchart illustrating processing performed in the present embodiment.
Fig. 13 is a flowchart illustrating processing performed in the present embodiment.
Fig. 14 is a flowchart illustrating processing performed in the present embodiment.
Fig. 15 is a diagram illustrating a schematic configuration of the image processing apparatus realized by installing, in a computer, an image processing program according to another embodiment.
Fig. 16 is a diagram illustrating a probability near a specific structure in a case where the specific structure is detected.
Fig. 17 is a diagram illustrating weights of the specific structure in a case where smoothing processing is performed.
Fig. 18 is a diagram illustrating a profile of pixel values of the specific structure before smoothing processing is performed.
Fig. 19 is a diagram illustrating a profile of pixel values of the specific structure after smoothing processing is performed.

### DETAILED DESCRIPTION

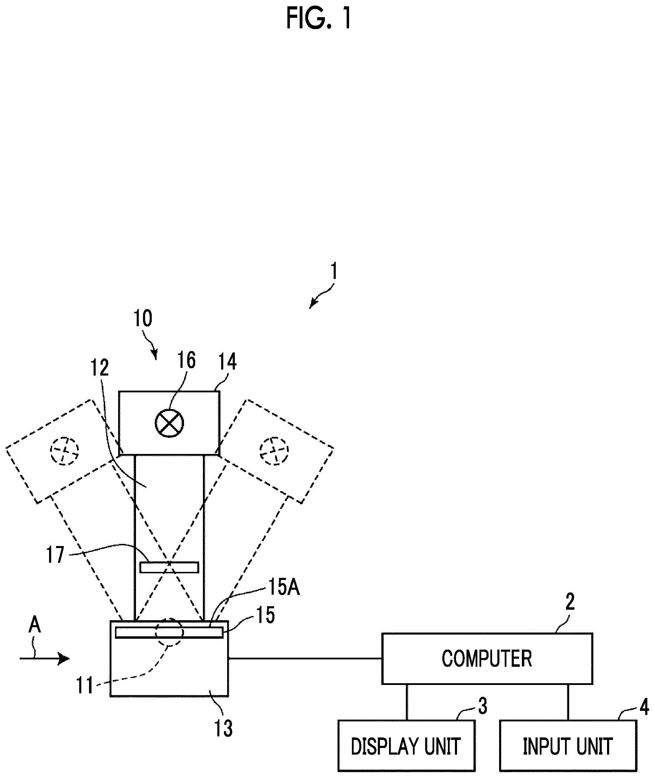

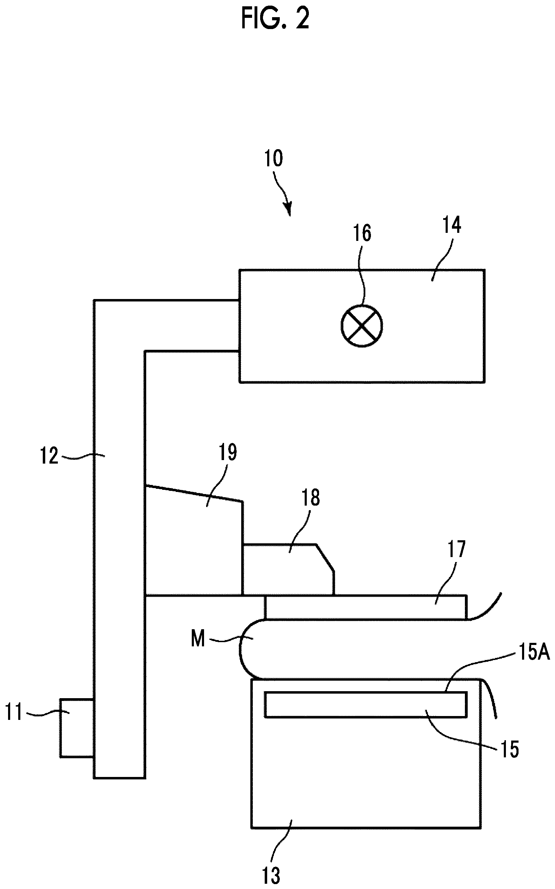

Hereinafter, embodiments of the present disclosure will be described with reference to the drawings. Fig. 1 is a schematic configuration diagram of a radiography apparatus to which an image processing apparatus according to an embodiment of the present disclosure is applied, and Fig. 2 is a diagram illustrating the radiography apparatus as viewed from a direction of an arrow A in Fig. 1. The radiography apparatus 1 is a mammography imaging apparatus that acquires a plurality of radiographic images, that is, a plurality of projection images by imaging a breast M as a subject at a plurality of radiation source positions in order to generate a tomographic image by performing tomosynthesis imaging of the breast. As illustrated in Fig. 1, the radiography apparatus 1 includes an imaging unit 10, a computer 2 connected to the imaging unit 10, and a display unit 3 and an input unit 4 connected to the computer 2.

The imaging unit 10 includes an arm portion 12 that is connected to a base (not illustrated) by a rotation shaft 11. An imaging table 13 is attached to one end of the arm portion 12, and a radiation irradiation unit 14 is attached to the other end of the arm portion 12 so as to face the imaging table 13. The arm portion 12 is configured such that only the end to which the radiation irradiation unit 14 is attached can be rotated. Therefore, the imaging table 13 is fixed and only the radiation irradiation unit 14 can be rotated. The rotation of the arm portion 12 is controlled by the computer 2.

A radiation detector 15, such as a flat panel detector, is provided in the imaging table 13. The radiation detector 15 has a radiation detection surface 15A. In addition, a circuit board including a charge amplifier that converts a charge signal read from the radiation detector 15 into a voltage signal, a sampling two correlation pile circuit that samples the voltage signal output from the charge amplifier, and an analog-to-digital (AD) conversion unit that converts the voltage signal into a digital signal is provided in the imaging table 13. The radiation detector 15 corresponds to a detection unit. Further, in the present embodiment, as the detection unit, the radiation detector 15 is used. On the other hand, the detection unit is not limited to the radiation detector 15 as long as the detection unit can detect radiation and convert the radiation into an image.

The radiation detector 15 can repeatedly perform recording and reading of a radiographic image, may be a so-called direct-type radiation detector that directly converts radiation such as X-rays into charges, or may be a so-called indirect-type radiation detector that converts radiation into visible light once and converts the visible light into a charge signal. As a method for reading a radiographic image signal, it is desirable to use the following method: a so-called thin film transistor (TFT) reading method which reads a radiographic image signal by turning on and off a TFT switch; or a so-called optical reading method which reads a radiographic image signal by irradiating a target with read light. On the other hand, the reading method is not limited thereto, and other methods may be used.

A radiation source 16 is accommodated in the radiation irradiation unit 14. The radiation source 16 emits X-rays as radiation. The computer 2 controls a timing when the radiation source 16 emits the radiation and radiation generation conditions of the radiation source 16, that is, selection of a target and filter materials, a tube voltage, an irradiation time, and the like.

Further, the arm portion 12 is provided with a compression plate 17 that is disposed above the imaging table 13 and presses and compresses the breast M, a support portion 18 that supports the compression plate 17, and a movement mechanism 19 that moves the support portion 18 in a vertical direction in Fig. 1 and Fig. 2. A distance between the compression plate 17 and the imaging table 13, that is, a compression thickness is input to the computer 2.

The display unit 3 is a display device such as a cathode ray tube (CRT) or a liquid crystal monitor, and displays a projection image, a tomographic image, a composite two-dimensional image, which are acquired as described later, and messages required for operations. The display unit 3 may include a speaker that outputs sound.

The input unit 4 includes a keyboard, a mouse, and a touch-panel-type input device, and receives an operation of the radiography apparatus 1 by the operator. Further, the input unit 4 receives an input of various kinds of information required for tomosynthesis imaging, such as imaging conditions, and an instruction to correct information. In the present embodiment, each unit of the radiography apparatus 1 is operated according to the information which is input from the input unit 4 by the operator.

An image processing program according to the present embodiment is installed on the computer 2. In the present embodiment, the computer 2 may be a workstation or a personal computer that is directly operated by the operator, or may be a server computer that is connected to the mammography apparatus 10 via a network. The image processing program is distributed by being recorded on a recording medium such as a digital versatile disc (DVD) or a compact disc read only memory (CD-ROM), and is installed in a computer from the recording medium. Alternatively, the image processing program is stored in a storage device of a server computer connected to the network or a network storage in a state where an access from the outside is allowed, and is downloaded and installed in the computer as required.

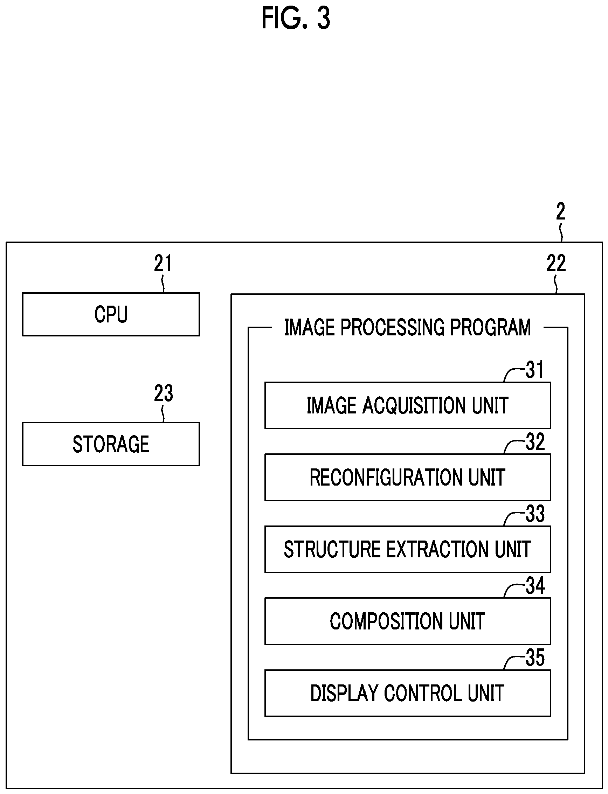

Fig. 3 is a diagram illustrating a schematic configuration of the image processing apparatus realized by installing, on the computer 2, the image processing program according to the present embodiment. As illustrated in Fig. 3, the image processing apparatus includes, as a standard computer configuration, a central processing unit (CPU) 21, a memory 22, and a storage 23.

The storage 23 is a storage device such as a hard disk drive or a solid state drive (SSD), and stores various kinds of information including a program for driving each unit of the radiography apparatus 1 and the image processing program. Further, the storage 23 also stores the projection image acquired by tomosynthesis imaging, the tomographic image generated as described later, and a composite two-dimensional image.

The memory 22 temporarily stores the programs, which are stored in the storage 23, in order to cause the CPU 21 to execute various processing. The image processing program define the following processing as processing to be executed by the CPU 21: image acquisition processing of acquiring a plurality of projection images of the breast M corresponding to each of a plurality of radiation source positions by causing the radiography apparatus 1 to perform tomosynthesis imaging; reconfiguration processing of generating a plurality of tomographic images on each of a plurality of tomographic planes of the breast M as a subject by reconfiguring the plurality of projection images; structure extraction processing of extracting a specific structure from each of the plurality of tomographic images; composition processing of generating a composite two-dimensional image from the plurality of tomographic images by setting a weight of a pixel of the specific structure in each of the plurality of tomographic images to be larger than a weight of a pixel other than the pixel of the specific structure; and display control processing of displaying the composite two-dimensional image or the like on the display unit 3.

The CPU 21 executes the processing according to the image processing program, and thus the computer 2 functions as an image acquisition unit 31, a reconfiguration unit 32, a structure extraction unit 33, a composition unit 34, and a display control unit 35.

In a case where image acquisition processing is performed, the image acquisition unit 31 acquires a plurality of projection images Gi (i = 1 to n, where n is the number of radiation source positions and is, for example, n=15) at a plurality of radiation source positions by moving the radiation source 16 by rotating the arm portion 12 around the rotation shaft 11, irradiating the breast M as a subject with radiation at a plurality of radiation source positions obtained by the movement of the radiation source 16 according to predetermined imaging conditions for tomosynthesis imaging, and detecting the radiation passing through the breast M by the radiation detector 15.

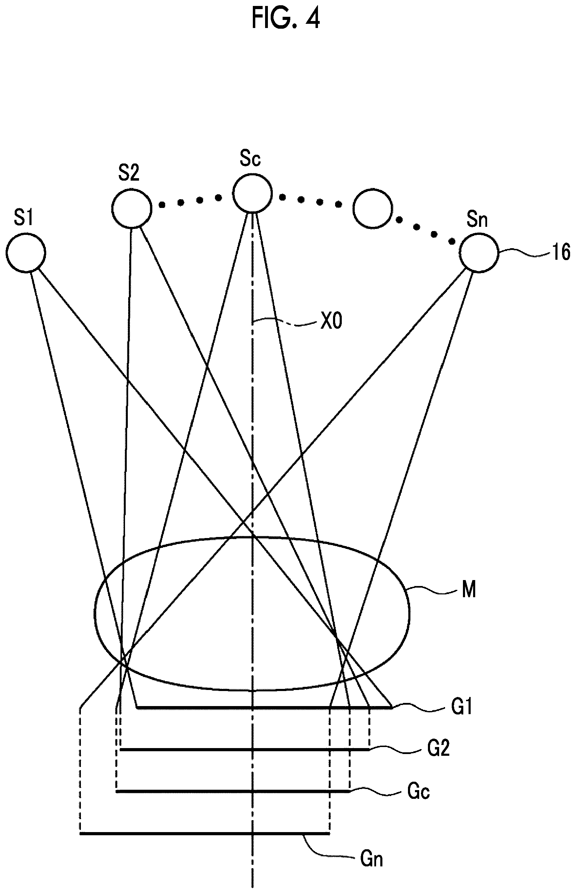

Fig. 4 is a diagram for explaining the acquisition of the projection images Gi. As illustrated in Fig. 4, the radiation source 16 is moved to each of radiation source positions S1, S2, ···, and Sn. The radiation source 16 drives and irradiates the breast M with radiation at each of the radiation source positions. The radiation detector 15 detects X-rays passing through the breast M, and thus the projection images G1, G2, ···, and Gn corresponding to the radiation source positions S1 to Sn are acquired. At each of the radiation source positions S1 to Sn, the breast M is irradiated with the same dose of radiation. The plurality of acquired projection images Gi are stored in the storage 23. Further, the plurality of projection images Gi may be acquired according to a program different from the image processing program, and may be stored in the storage 23 or an external storage device. In this case, the image acquisition unit 31 reads the plurality of projection images Gi from the storage 23 or the external storage device for reconfiguration processing or the like, the projection images Gi being stored in the storage 23 or the external storage device.

In Fig. 4, the radiation source position Sc is a radiation source position at which the optical axis X0 of the radiation emitted from the radiation source 16 is orthogonal to the detection surface 15A of the radiation detector 15. The radiation source position Sc is referred to as a reference radiation source position Sc.

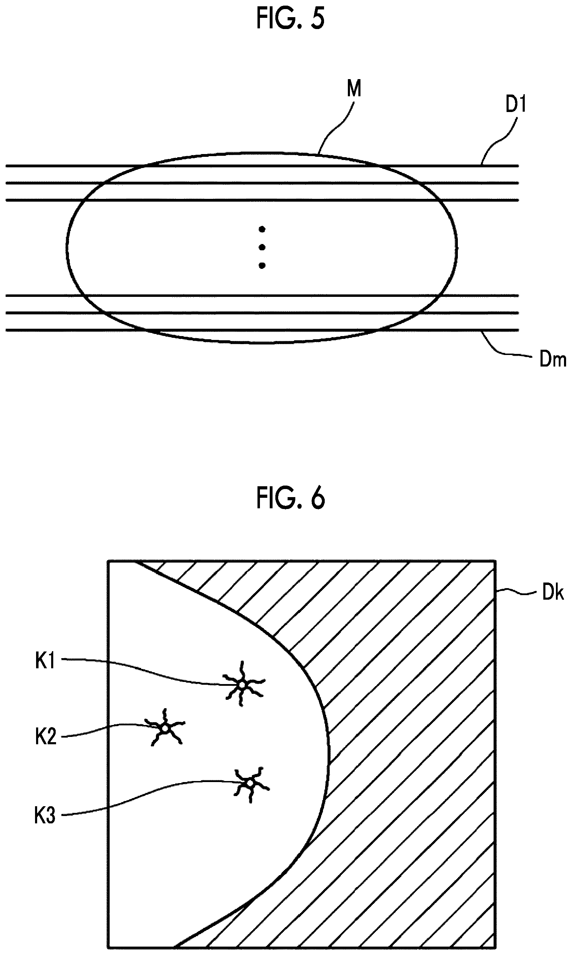

The reconfiguration unit 32 generates tomographic images in which the desired tomographic planes of the breast M are highlighted by reconfiguring the plurality of projection images Gi. Specifically, the reconfiguration unit 32 generates a plurality of tomographic images Dj (j = 1 to m) on each of the plurality of tomographic planes of the breast M as illustrated in Fig. 5 by reconfiguring the plurality of projection images Gi using a known inverse projection method, such as a simple inverse projection method or a filtering inverse projection method. In this case, a three-dimensional coordinate position in a three-dimensional space including the breast M is set, pixel values at corresponding pixel positions in the plurality of projection images Gi are reconfigured with respect to the set three-dimensional coordinate position, and pixel values at the coordinate positions are calculated.

The structure extraction unit 33 extracts a specific structure from the plurality of tomographic images Dj. In the present embodiment, a spicula and a tumor included in the breast M are extracted as specific structures. Fig. 6 is a diagram for explaining extraction of a specific structure. Here, detection of a specific structure from one tomographic image Dk among the plurality of tomographic images Dj will be described. As illustrated in Fig. 6, the tomographic image Dk includes, as specific structures K1 to K3, a spicula and a tumor on the tomographic plane of the breast M on which the tomographic image Dk is acquired. The specific structure includes a spicula as a linear structure around a tumor.

The structure extraction unit 33 extracts a specific structure from the tomographic image Dk by using a known computer aided diagnosis (hereinafter, referred to as CAD) algorithm. By using the CAD algorithm, a probability indicating that a pixel in the tomographic image Dj corresponds to a specific structure is derived, and in a case where the probability is equal to or higher than a predetermined threshold value, the pixel is detected as a specific structure. A method of extracting a specific structure is not limited to the method using CAD. A method of extracting a specific structure from the tomographic image Dk by performing filtering processing using a filter so as to extract a specific structure.

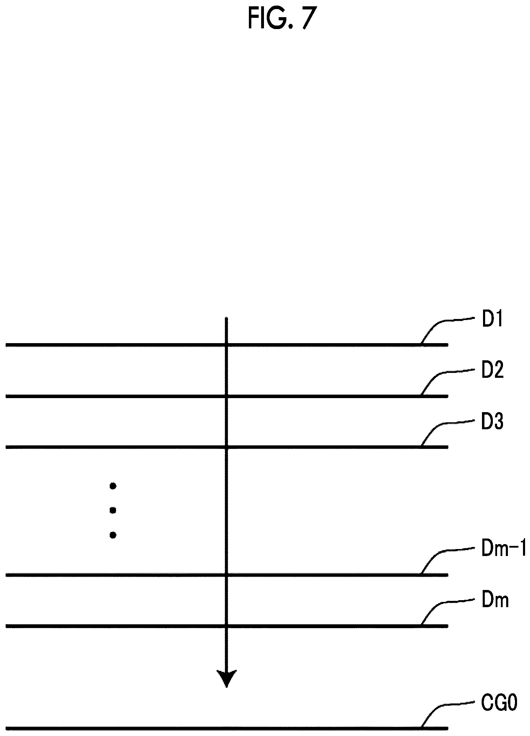

The composition unit 34 generates a composite two-dimensional image CG0, which is a pseudo two-dimensional image corresponding to a simple two-dimensional image obtained by irradiating the breast M with radiation at the reference radiation source position Sc and imaging the breast M, using the plurality of tomographic images Dj. In the present embodiment, the composition unit 34 generates a composite two-dimensional image CG0 by using an addition method, which weights and adds a pixel value of a corresponding pixel in each tomographic image Dj along a viewing direction from the reference radiation source position Sc toward the radiation detector 15, that is, along an optical axis X0 illustrated in Fig. 4, in a state where the plurality of tomographic images Dj are stacked as illustrated in Fig. 7. A method of generating the composite two-dimensional image CG0 is not limited to the addition method, and a known technique may be applied. In the addition method, in a state where the number of the tomographic images Dj is m, a weight for each pixel in a case of weighting and adding the pixel values is basically set to 1/m, and a composite two-dimensional image CG0 is generated. On the other hand, in the present embodiment, a weight of a pixel of the specific structure in each of the plurality of tomographic images Dj is set to be larger than a weight of a pixel other than the pixel of the specific structure, and a composite two-dimensional image CG0 is generated.

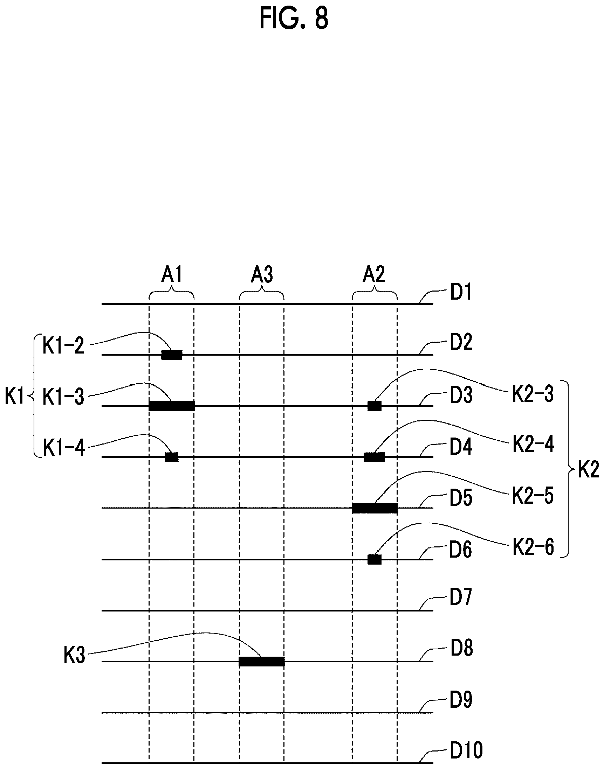

Fig. 8 is a diagram for explaining setting of weights in the present embodiment. In Fig. 8, for explanation, it is assumed that 10 tomographic images D1 to D10 are generated, a specific structure K1 is included in the tomographic images D2 to D4, a specific structure K2 is included in the tomographic images D3 to D6, and a specific structure K3 is included in the tomographic image D8. Although the specific structures K1 to K3 have a thickness in Fig. 8 for the sake of explanation, the specific structures K1 to K3 do not actually have a thickness. In addition, it is assumed that the specific structure K1 included in each of the tomographic images D2 to D4 includes specific structures K1-2, K1-3, and K1-4. Further, it is assumed that the specific structure K2 included in each of the tomographic images D3 to D6 includes specific structure K2-3, K2-4, K2-5, and K2-6.

The specific structure K1 illustrated in Fig. 8 is included over three tomographic images D2 to D4. Further, the specific structure K1-3 included in the tomographic image D3 has a largest size. In the tomographic images D1, and D5 to D10 that do not include the specific structure K1, the composition unit 34 sets a weight of a corresponding pixel corresponding to the specific structure K1 to be smaller than a weight of a pixel other than the corresponding pixel. Specifically, the composition unit 34 sets a weight of a corresponding pixel, which is a pixel in a corresponding region A1 on the tomographic images D1, and D5 to D10 corresponding to the specific structure K1 illustrated in Fig. 8, to be smaller than a weight of a pixel in a region other than the corresponding region A1. In the present embodiment, the weight of the corresponding pixel in the corresponding region A1 on the tomographic images D1, and D5 to D10 is set to 0. Further, in the tomographic image D2, a region of the specific structure K1-2 is smaller than the corresponding region A1. Thus, the composition unit 34 sets a weight of a pixel in a region other than the specific structure K1-2 in the corresponding region A1 of the tomographic image D2 to 0. Further, in the tomographic image D4, a region of the specific structure K1-4 is smaller than the corresponding region A1. Thus, the composition unit 34 sets a weight of a pixel in a region other than the specific structure K1-4 in the corresponding region A1 of the tomographic image D4 to 0. The processing of setting the weight will be described later.

The specific structure K2 illustrated in Fig. 8 is included over four tomographic images D3 to D6. Further, the specific structure K2-5 included in the tomographic image D5 has a largest size. In the tomographic images D1, D2, and D7 to D10 that do not include the specific structure K2, the composition unit 34 sets a weight of a corresponding pixel corresponding to the specific structure K2 to be smaller than a weight of a pixel other than the corresponding pixel. Specifically, the composition unit 34 sets a weight of a corresponding pixel, which is a pixel in a corresponding region A2 on the tomographic images D1, D2, and D7 to D10 corresponding to the specific structure K2 illustrated in Fig. 8, to 0. Further, in the tomographic image D3, a region of the specific structure K2-3 is smaller than the corresponding region A2. Thus, the composition unit 34 sets a weight of a pixel in a region other than the specific structure K2-3 in the corresponding region A2 of the tomographic image D3 to 0. Further, in the tomographic image D4, a region of the specific structure K2-4 is smaller than the corresponding region A2. Thus, the composition unit 34 sets a weight of a pixel in a region other than the specific structure K2-4 in the corresponding region A2 of the tomographic image D4 to 0. Further, in the tomographic image D6, a region of the specific structure K2-6 is smaller than the corresponding region A2. Thus, the composition unit 34 sets a weight of a pixel in a region other than the specific structure K2-6 in the corresponding region A2 of the tomographic image D6 to 0.

The specific structure K3 illustrated in Fig. 8 is included in only one tomographic image D8. In the tomographic images D1 to D7, D9, and D10 that do not include the specific structure K3, the composition unit 34 sets a weight of a corresponding pixel corresponding to the specific structure K3 to be smaller than a weight of a pixel other than the corresponding pixel. That is, the composition unit 34 sets a weight of a corresponding pixel in a corresponding region A3 on the tomographic images D1 to D7, D9, and D10 corresponding to the specific structure K3 illustrated in Fig. 8, to 0.

On the other hand, the composition unit 34 sets, for pixels of the specific structures K1 to K3 in each of the plurality of tomographic images Dj, a weight to 1. Thereby, a total value of weights of the pixels of the specific structures in the composite two-dimensional image CG0 is the number of the pixels of the specific structures included in the corresponding pixels. That is, a pixel value of the specific structure in the composite two-dimensional image CG0 is a sum of the pixel values of the specific structures in each of the tomographic images Dj.

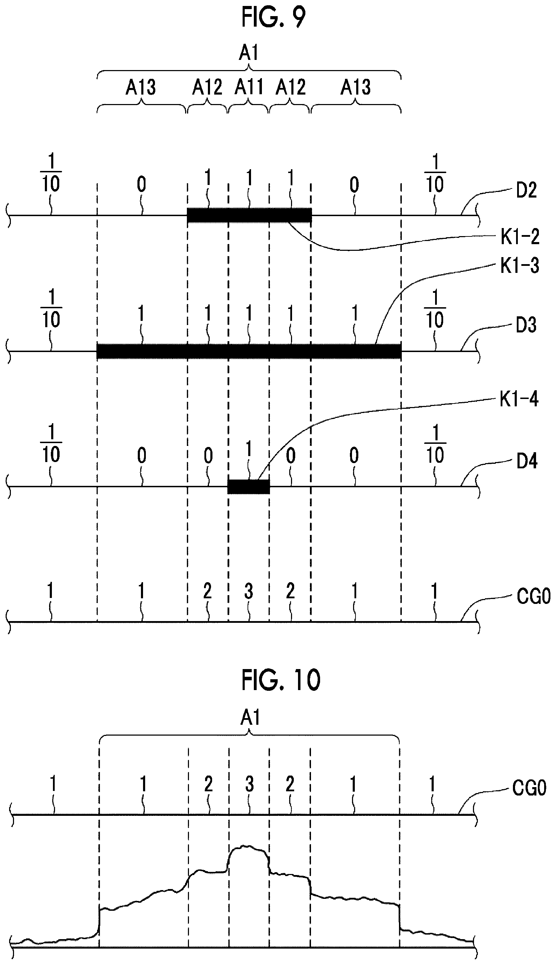

Fig. 9 is a diagram for explaining setting of weights for the specific structures. Although Fig. 9 illustrates setting of weights for the specific structure K1, weights may be set for the specific structures K2 and K3 in the same manner. As illustrated in Fig. 9, the composition unit 34 sets, for a pixel in a region of the specific structure K1 in the tomographic images D2 to D4, a weight to 1. Here, in the tomographic images D1, and D5 to D10, a weight of a pixel in the corresponding region A1 is set to 0. Thus, on the composite two-dimensional image CG0, among the pixels in the corresponding region A1 corresponding to the specific structure K1, for pixels in a region A11 in which all three specific structures K1-2, K1-3, and K1-4 overlap with each other, a total value of the weights is 3.

Further, on the composite two-dimensional image CG0, among the pixels in the corresponding region A1, for pixels in a region A12 in which only two specific structures K1-2 and K1-3 in the tomographic images D2 and D3 overlap with each other, a total value of the weights is 2.

Further, on the composite two-dimensional image CG0, among the pixels in the corresponding region A1, for pixels in a region A13 in which the specific structure K1-3 in the tomographic image D3 does not overlap with the specific structures K1-2 and K1-4 of other tomographic images D2 and D4, a total value of the weights is 1.

In addition, in regions other than the corresponding regions A1 of the tomographic images D2 to D4, for pixels in the regions that do not overlap with any specific structure included in other tomographic images D1, and D5 to D10, the number of the tomographic images is 10. For this reason, in consideration of a noise pixel to be described, the composition unit 34 sets a weight of a pixel in the regions to 1/10. Therefore, on the composite two-dimensional image CG0, for pixels in the regions that do not overlap with any specific structure, a total value of the weights is 1.

In the present embodiment, the composition unit 34 sets the weights as described above, and thus, the weight of the pixel of the specific structure included in each tomographic image Dj is set to be larger than the weights of the pixels other than the pixel of the specific structure. Here, a pixel value of the specific structure is larger than a pixel value of a structure other than the specific structure. Therefore, in the composite two-dimensional image CG0 generated by using the weights which are set as described above, as illustrated in Fig. 10, a pixel value of a pixel in the corresponding region A10 has a larger value than a pixel value of a pixel in other regions.

Further, in the present embodiment, for a pixel which is greatly influenced by a noise, such as a pixel with a high proportion of noise components (hereinafter, referred to as a noise pixel) in regions other than a region of a specific structure and regions other than the corresponding region corresponding to the specific structure (hereinafter, referred to as other regions) in each tomographic image Dj, the composition unit 34 lowers a weight of the noise pixel or excludes the noise pixel in generation of the composite two-dimensional image CG0. Excluding the noise pixel means setting the weight of the noise pixel to 0. In the present embodiment, it is assumed that the weight for the noise pixel is set to 0.

Here, in the tomographic image Dj, the pixel of the specific structure has a large signal according to the specific structure. On the other hand, a pixel other than the pixel of the specific structure tends to have a relatively small signal although a noise constantly occurs and an influence of the noise appears in the signal. For this reason, in the present embodiment, the composition unit 34 derives, in other regions of the plurality of tomographic images Dj, an average value of pixel values of all the pixels which are added in a case where the composite two-dimensional image CG0 is generated, considers, as a noise pixel, a pixel in which an absolute value of a value obtained by dividing the average value by each pixel value is equal to or smaller than a predetermined threshold value Th, and sets a weight for the noise pixel to 0. The threshold value Th may be determined in advance by an experiment, a simulation, or the like according to a magnitude of the superimposed noise.

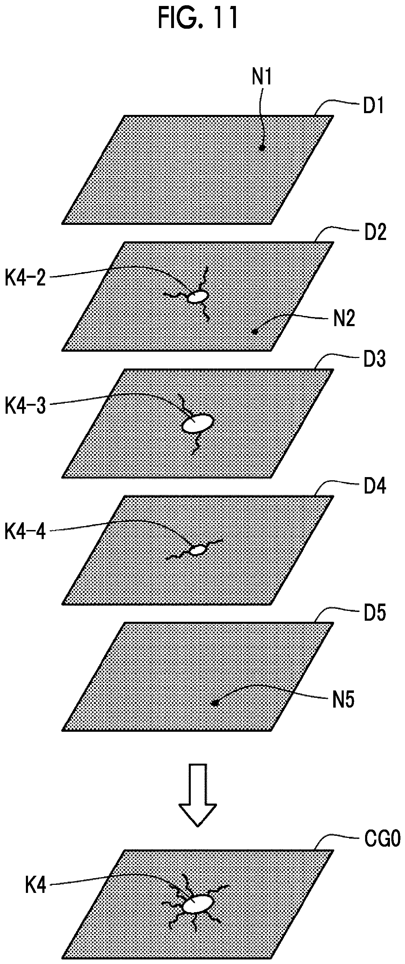

The composition unit 34 sets the weights as described above. Thereby, for the regions including the specific structures in the tomographic images Dj, a composite two-dimensional image CG0 is generated as follows. Fig. 11 is a diagram for explaining generation of a composite two-dimensional image. In Fig. 11, for the sake of explanation, an example of generating a composite two-dimensional image CG0 from five tomographic images D1 to D5 will be described. As illustrated in Fig. 11, it is assumed that the tomographic images D2 to D4 among the tomographic images D1 to D5 respectively include specific structures K4-2, K4-3, and K4-4 representing the same specific structure. In the specific structures K4-2, K4-3, and K4-4, a spicula as a linear structure is present around a tumor. Further, the tomographic images D1, D2, and D5 respectively include noises N1, N2, and N5.

As described above, the weights of the pixels of the specific structures K4-2, K4-3, and K4-4 are set to be larger than the weights of the pixels in the regions other than the specific structures K4-2, K4-3, and K4-4. In particular, for the specific structures K4-2, K4-3, and K4-4, the weights are respectively set to 1. Further, the weights of the pixels in the corresponding regions corresponding to the specific structures K4-2, K4-3, and K4-4 in the tomographic images D1 and D5 are set to 0. Thus, a signal value of the pixel of the specific structure K4 in the composite two-dimensional image CG0 is a value obtained by adding only signal values of the specific structures K4-2, K4-3, and K4-4 included in the tomographic images D2 to D4. Therefore, in the composite two-dimensional image CG0, the specific structure is not hidden in other regions.

On the other hand, for the pixels in the regions other than the specific structures K4-2, K4-3, and K4-4 and the corresponding regions corresponding to the specific structures K4-2, K4-3, and K4-4 in the tomographic images D1 to D5, the weights are set to 1/5. Further, for the noise pixel, the weight is set to 0 in a case where a composite two-dimensional image is generated. Thereby, other regions other than the specific structure K4 in the composite two-dimensional image CG0 have substantially the same signal values as the pixels in the regions other than the specific structure in the tomographic images D1 to D5, and thus the noise is removed.

In other regions of each tomographic image Dj, for pixels including the noise pixels, the weight in addition is set based on a value obtained by subtracting the number of the noise pixels from the number of the tomographic images. That is, during a period for which a composite two-dimensional image CG0 is generated by adding m tomographic images, in a case where there are d noise pixels in the tomographic images Dj corresponding to a certain pixel on the composite two-dimensional image CG0, the weight for the noise pixels is set to 1/(m-d) instead of 1/m.

As described above, in a case where the weight for the region of the specific structure in each tomographic image Dj is set to 1, the pixel value of the specific structure on the composite two-dimensional image CG0 may exceed a maximum value of the pixel value that is allowed for the composite two-dimensional image CG0. For example, in Fig. 9, for the pixels in the region A11 in which all the specific structures K1-2, K1-3, and K1-4 overlap with each other, a total value of the weights is 3. In this case, assuming that the maximum value of the pixel value which is allowed for the composite two-dimensional image CG0 is 1024 and that the pixel value of each of the specific structures K1-2, K1-3, and K1-4 is 512, the pixel value in the composite two-dimensional image CG0 is 1536. Thus, the maximum value of the pixel value which is allowed for the composite two-dimensional image CG0 is larger than 1024. As a result, the pixel is in a state where the pixel value is saturated. For this reason, in a case where the pixel value of the specific structure on the composite two-dimensional image CG0 is larger than the maximum value of the pixel value which is allowed for the composite two-dimensional image CG0, the composition unit 34 normalizes a pixel value of a pixel in the region of the specific structure in the composite two-dimensional image CG0 by the maximum value of the pixel value which is allowed for the composite two-dimensional image CG0.

For example, in a case where the pixel value in the composite two-dimensional image CG0 is 1536 and the pixel value corresponds to the maximum value of the pixel value in the specific structure, the pixel value in the specific structure is normalized so as to make the pixel value of 1536 to be 1024. In this case, the pixel value of all the pixels in the specific structure is normalized so as to be 1024/1536 times the pixel value. Thereby, it possible to prevent the pixel value in the specific structure included in the composite two-dimensional image CG0 from being saturated.

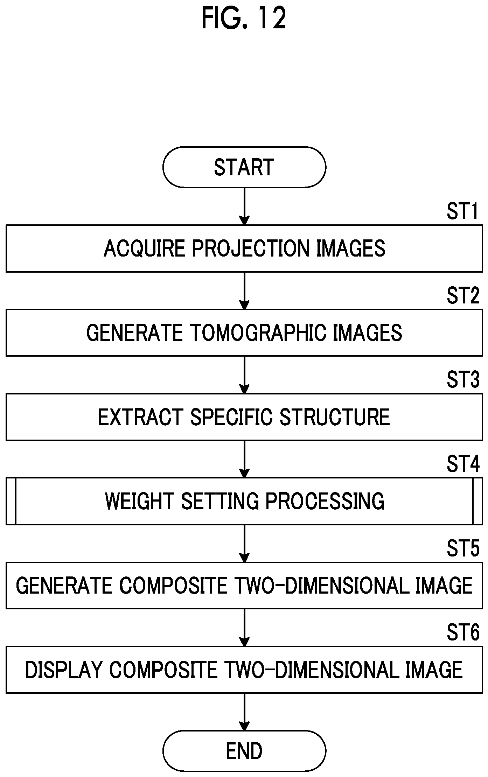

Next, processing performed in the present embodiment will be described. Fig. 12 is a flowchart illustrating processing performed in the present embodiment. In a case where the input unit 4 receives an instruction to start processing by the operator, tomosynthesis imaging is performed, and the image acquisition unit 31 acquires a plurality of projection images Gi (step ST1). Next, the reconfiguration unit 32 generates a plurality of tomographic images Dj on a plurality of tomographic planes of the breast M by reconfiguring the plurality of projection images Gi (step ST2). It is assumed that the number of the tomographic images is m. Further, the structure extraction unit 33 extracts a specific structure from each of the plurality of tomographic images Dj (step ST3). Next, the composition unit 34 sets the weight for each pixel of the plurality of tomographic images Dj in a case where a composite two-dimensional image CG0 is generated (weight setting processing, step ST4).

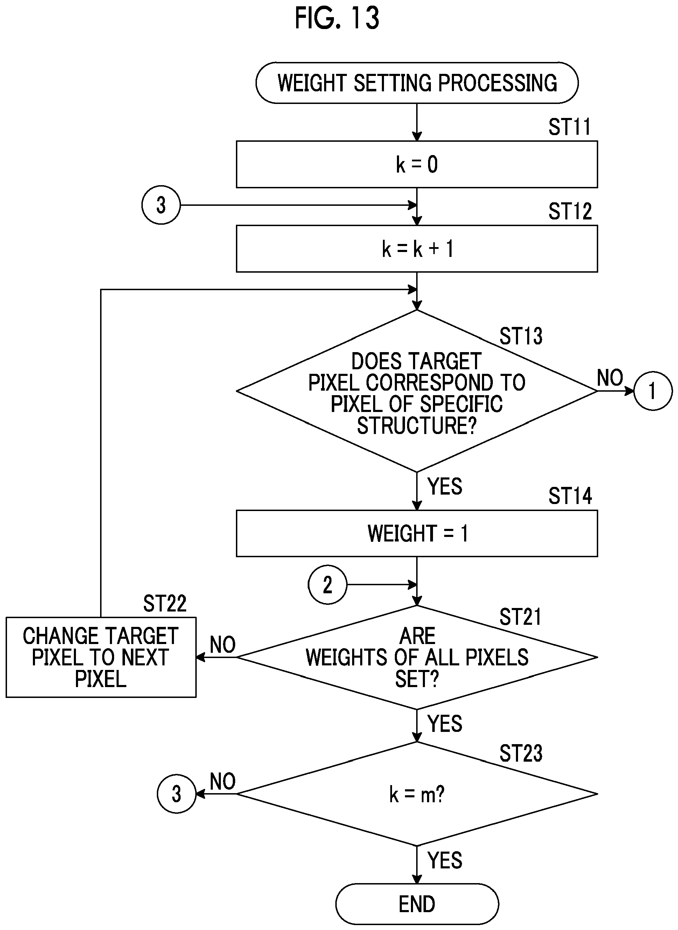

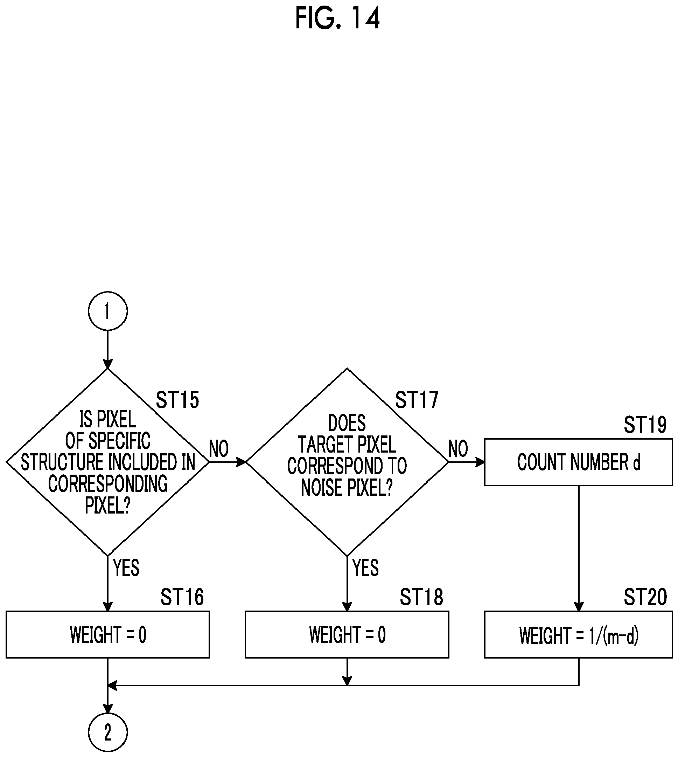

Fig. 13 and Fig. 14 are flowcharts of weight setting processing. In the weight setting processing, the composition unit 34 sets a variable k for managing the number of the tomographic images for which the weights are set to 0 (k=0, step ST11), and adds 1 to the variable k (step ST12). The composition unit 34 determines whether or not a target pixel in the k-th tomographic image corresponds to the pixel of the specific structure (step ST13). In a case where a determination result in step ST13 is YES, the composition unit 34 sets the weight of the target pixel to 1 (step ST14), and the process proceeds to step ST21.

On the other hand, in a case where a determination result in step ST13 is NO, the composition unit 34 determines whether or not the pixel of the specific structure in other tomographic images other than the k-th tomographic image is included in the corresponding pixel corresponding to the target pixel (step ST15). In a case where a determination result in step ST15 is YES, the composition unit 34 sets the weight of the target pixel to 0 (step ST16), and the process proceeds to step ST21. In a case where a determination result in step ST15 is NO, the composition unit 34 determines whether or not the target pixel is a noise pixel (step ST17). In a case where a determination result in step ST17 is YES, the composition unit 34 sets the weight of the target pixel to 0 (step ST18), and the process proceeds to step ST21. In a case where a determination result in step ST17 is NO, the composition unit 34 counts, in other tomographic images other than the k-th tomographic image, the number d of the tomographic images in which the corresponding pixel corresponding to the target pixel is a noise pixel (step ST19), sets the weight of the target pixel to 1/(m-d) (step ST20), and the process proceeds to step ST21.

In step ST21, the composition unit 34 determines whether or not the weights of all the pixels in the k-th tomographic image are set. In a case where a determination result in step ST21 is NO, the target pixel is changed to the next pixel (step ST22). Thereafter, the process returns to step ST13, and processing of step ST13 and the subsequent steps is repeated. In a case where a determination result in step ST21 is YES, the composition unit 34 determines whether or not the variable k is equal to the number m of the tomographic images (k=m) (step ST23). In a case where the variable k is equal to the number m of the tomographic images, that is, in a case where there is a tomographic image for which the weight of the target pixel is not set, a determination result in step ST23 is NO. Thereafter, the process returns to step ST12, and processing of step ST12 and the subsequent steps is repeated. In a case where a determination result in step ST23 is YES, the weight setting processing is ended.

Returning to Fig. 12, the composition unit 34 generates a composite two-dimensional image CG0 from the plurality of tomographic images Dk using the set weights (step ST5). At this time, in case of necessity, as described above, the pixel value of the pixel of the specific structure on the composite two-dimensional image CG0 is normalized by the maximum value of the pixel value of the composite two-dimensional image CG0. The display control unit 35 displays the composite two-dimensional image CG0 on the display unit 3 (step ST6), and the processing is ended.

As described above, in the present embodiment, the composite two-dimensional image CG0 is generated from the plurality of tomographic images Dj by extracting the specific structure from each of the plurality of tomographic images Dj representing the tomographic planes of the breast M, and setting the weight of the pixel of the specific structure in each of the plurality of tomographic images Dj to be larger than the weight of the pixel other than the pixel of the specific structure. Therefore, in the composite two-dimensional image CG0, the specific structure is not hidden in other regions other than the specific structure. Thereby, according to the present embodiment, it is possible to easily find the specific structure in the composite two-dimensional image CG0.

On the other hand, in the simple two-dimensional image of the breast that is acquired by simple imaging, structures such as a spicula and a tumor are represented in high brightness (white), and adipose tissues other than the structures are represented in low brightness (black). However, in the methods described in US8983156B and US2014/0327702A, in a case where a composite two-dimensional image CG0 is generated, signal values of the corresponding pixels in the tomographic images are averaged. As a result, a region of the adipose tissues included in the composite two-dimensional image CG0 is represented to be whitish as compared with the simple two-dimensional image. For this reason, a texture of the composite two-dimensional image CG0 is different from a texture of the simple two-dimensional image. Further, in the method described in US10140715B, in the tomographic images which are above and below a linear structure such as a spicula, edges such as normal mammary glands included in the tomographic images are detected. On the other hand, in the composite two-dimensional image CGO0, the edges such as mammary glands overlap with a spicula, and as a result, it is difficult to find the spicula. Further, in the method described in US10140715B, the detected edges such as mammary glands may overlap with each other, and as a result, a structure that looks like a spicula may appear in the composite two-dimensional image CG0.

In the present embodiment, in each of the plurality of tomographic images Dj, the weight of the corresponding pixel corresponding to the specific structure included in other tomographic images is set to be smaller than the weight of the pixel other than the corresponding pixel, preferably, to 0. Thereby, the specific structure included in the composite two-dimensional image CG0 can be less influenced by the pixels other than the pixel of the specific structure, for example, edges of mammary glands, in other tomographic images other than the tomographic image including the specific structure. Therefore, it is possible to make the composite two-dimensional image CG0 have the same texture as a texture of the simple two-dimensional image. Further, in the composite two-dimensional image CG0, the specific structure does not overlap with structures other than the specific structure. Thus, it is possible to prevent the specific structure from being difficult to find. Further, it is possible to prevent a structure which is different from the specific structure and looks like the specific structure from appearing in the composite two-dimensional image CG0.

In the above-described embodiment, the plurality of tomographic images are acquired by tomosynthesis imaging. The tomosynthesis imaging also has a problem that the reconfigured tomographic image is blurred due to an influence by a mechanical error of the imaging apparatus, a body movement of the subject due to a time difference of imaging at each of a plurality of radiation source positions, or the like. In a case where the tomographic image is blurred in this way, it is difficult to detect a lesion such as minute calcification which is useful for early detection of breast cancer.

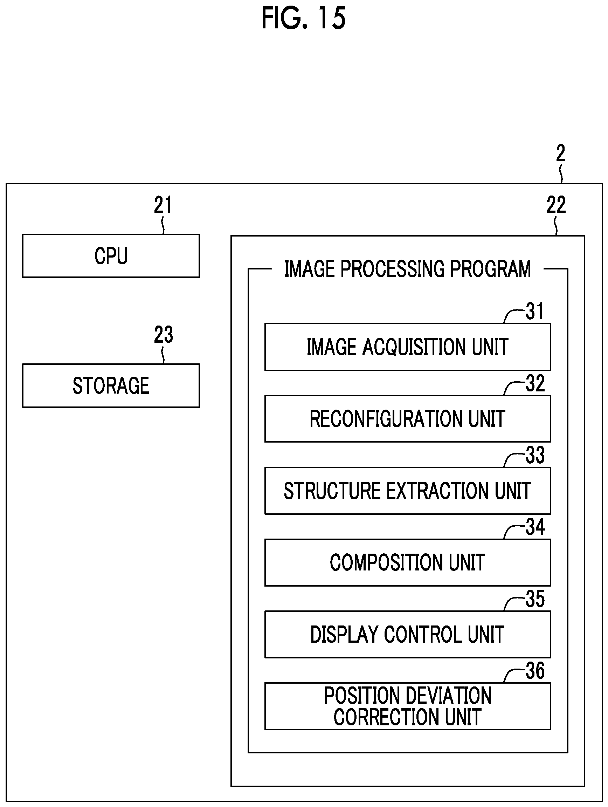

For this reason, as illustrated in the image processing apparatus according to another embodiment illustrated in Fig. 15, a position deviation correction unit 36 for correcting a position deviation due to a body movement or the like in a plurality of projection images acquired by tomosynthesis imaging may be provided. For the position deviation correction by the position deviation correction unit 36, any method such as a method described in JP2016-064119A may be used. The method described in JP2016-064119A is a method for correcting a position deviation in a plurality of projection images Gi such that positions in the plurality of projection images Gi match with each other. Specifically, there is disclosed a method of correcting a position deviation due to a body movement or the like by detecting feature points included in each projection image Gi, such as edges, edge intersections, and edge corners, using an algorithm such as scale-invariant feature transform (SIFT) or speeded up robust features (SURF) and transforming each projection image Gi such that the detected feature points match with each other.

In this way, in a case where the tomographic images are generated by performing position deviation correction and reconfiguring the projection images, the minute calcification included in the breast M does not disappear in the tomographic images. Therefore, in the composite two-dimensional image CG0, an abnormal portion can be completely expressed.

Further, in the above-described embodiment, the composition unit may perform smoothing processing on a boundary of the specific structure in a case where the composite two-dimensional image CG0 is generated. The smoothing processing means processing of matching a pixel value near a boundary of the specific structure with a pixel value of a background in contact with the boundary of the specific structure by smoothly changing the pixel value near the boundary of the specific structure. Specifically, in a case where the composite two-dimensional image CG0 is generated, the smoothing processing is performed by changing the weight of the pixel near the boundary of the specific structure. The weight in the smoothing processing may be set based on a probability in a case where the structure extraction unit 33 detects the specific structure.

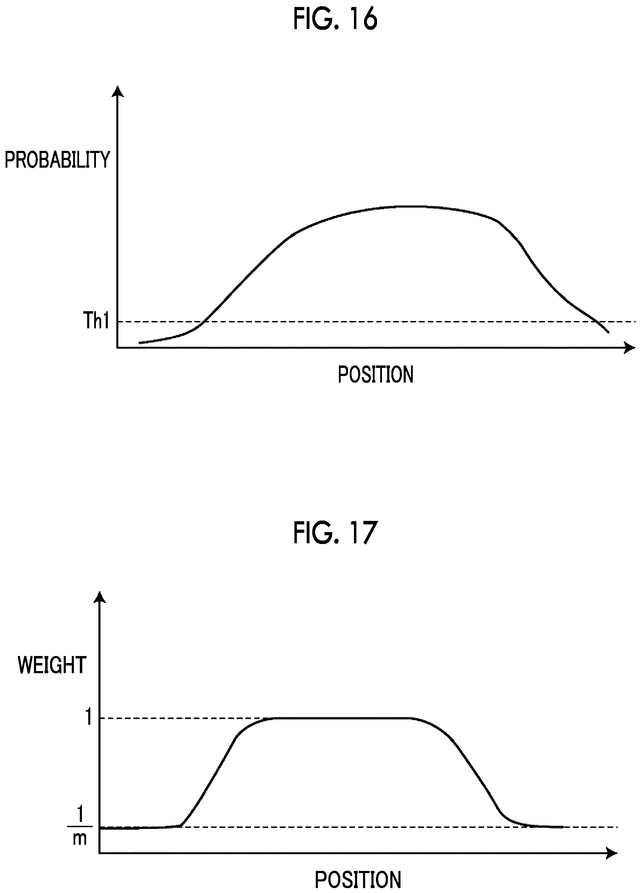

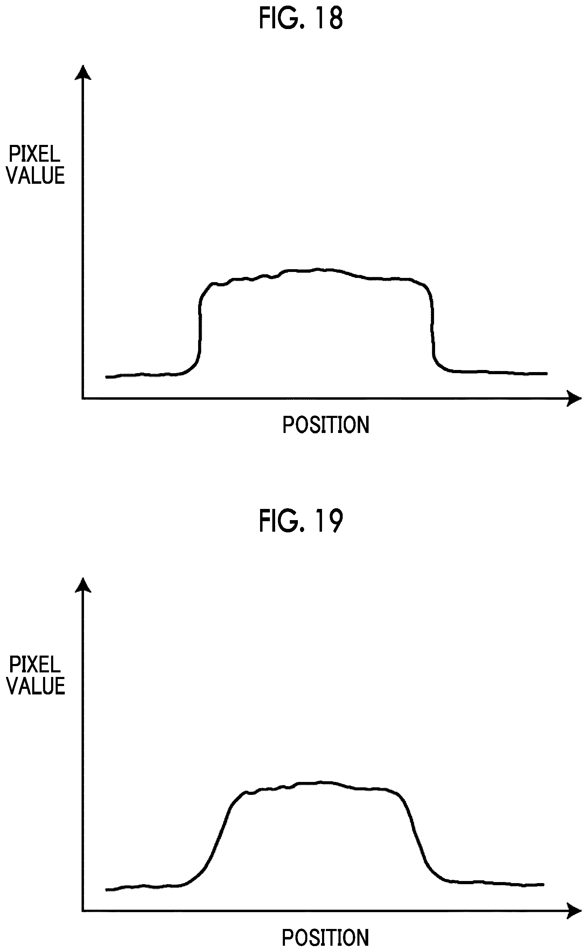

Here, Fig. 16 illustrates a distribution of the probability output by the structure extraction unit 33. As illustrated in Fig. 16, the probability output by the structure extraction unit 33 becomes lower as the pixel is away from the specific structure, and a region in which the probability is equal to or higher than a threshold value Th1 is detected as the specific structure. Therefore, as illustrated in Fig. 17, the composition unit 34 sets the weight for the specific structure in accordance with the probability of the specific structure such that the weight gradually decreases from 1 to 1/m as the pixel is away from the specific structure. Thereby, the specific structure illustrated in Fig. 18 has an unnaturally clear pixel value at a boundary with neighbor pixels, whereas the specific structure illustrated in Fig. 19 has a pixel value which is smoothly changed at the boundary with neighbor pixels. Thus, in the composite two-dimensional image CG0, the boundary of the specific structure gradually approaches a pixel value of a background to which the pixel value of the specific structure is adjacent. Therefore, it is possible to generate the composite two-dimensional image CG0 in which the specific structure and the background of the specific structure more naturally harmonize with each other.

The smoothing processing is not limited to the method using the weights derived as described above. The smoothing processing may be performed by filtering the boundary of the specific structure included in the composite two-dimensional image CG0 by using a low-pass filter.

Further, in the above-described embodiment, in the plurality of tomographic images Dj, it is determined whether or not the pixel in the specific structure and the corresponding region corresponding to the specific structures of other tomographic images is a noise pixel. On the other hand, the present disclosure is not limited thereto. Without determining whether or not the pixel is a noise pixel, a value 1/m obtained by dividing 1 by the number m of the tomographic images may be set as a weight.

Further, in the above-described embodiment, in each of the tomographic images Dj, the weight of the specific structure is set to 1. On the other hand, the weight is not limited thereto. In the plurality of tomographic images Dj, assuming that the number of the pixels of the specific structure included in the corresponding pixels is c, the weights of the pixels of the specific structure may be set to 1/c. In this case, the composition unit 34 sets the weights of the pixels of the specific structure such that a total value of the weights is 1 in a case where the composite two-dimensional image CG0 is generated.

Further, in the above-described embodiment, the addition method is applied as the method for generating the composite two-dimensional image in the composition unit 34. On the other hand, as described above, another known technique may be applied. For example, a so-called minimum path method, which uses a minimum value of the corresponding pixel of each tomographic image, may be applied.

Further, in the above-described embodiment, the radiation is not particularly limited. For example, α-rays or γ-rays other than X-rays may be applied.

Further, in the above-described embodiment, for example, the following various processors may be used as a hardware structure of processing units performing various processing, such as the image acquisition unit 31, the reconfiguration unit 32, the structure extraction unit 33, the composition unit 34, the display control unit 35, and the position deviation correction unit 36. The various processors include, as described above, a CPU, which is a general-purpose processor that functions as various processing units by executing software (program), and a dedicated electric circuit, which is a processor having a circuit configuration specifically designed to execute a specific processing, such as a programmable logic device (PLD) or an application specific integrated circuit (ASIC) that is a processor of which the circuit configuration may be changed after manufacturing such as a field programmable gate array (FPGA).

One processing unit may be configured by one of these various processors, or may be configured by a combination of two or more processors having the same type or different types (for example, a combination of a plurality of FPGAs or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Further, as the hardware structure of the various processors, more specifically, an electric circuit (circuitry) in which circuit elements such as semiconductor elements are combined may be used.

### Explanation of References

- 1:: radiography apparatus
- 2:: computer
- 3:: display unit
- 4:: input unit
- 10:: imaging unit
- 15:: radiation detector
- 16:: radiation source
- 17:: compression plate
- 21:: CPU
- 22:: memory
- 23:: storage
- 31:: image acquisition unit
- 32:: reconfiguration unit
- 33:: structure extraction unit
- 34:: composition unit
- 35:: display control unit
- 36:: position deviation correction unit
- A1 to A3:: corresponding region
- CG0:: composite two-dimensional image
- Dj (j = 1 to m), Dk:: tomographic image
- Gi (i = 1 to n):: projection image
- K1 to K4:: specific structure
- M:: breast
- Si (i = 1 to n):: radiation source position
- Sc:: reference radiation source position
- X0:: optical axis

## Claims

1. An image processing apparatus comprising:
a structure extraction unit that extracts a specific structure from each of a plurality of tomographic images representing a plurality of tomographic planes of a subject; and
a composition unit that generates a composite two-dimensional image from the plurality of tomographic images by setting a weight of a pixel of the specific structure in each of the plurality of tomographic images to be larger than a weight of a pixel other than the pixel of the specific structure.

2. The image processing apparatus according to claim 1,
wherein the composition unit sets, in pixels other than the pixel of the specific structure in each of the plurality of tomographic images, a weight of a corresponding pixel corresponding to the specific structure included in other tomographic images to be smaller than a weight of a pixel other than the corresponding pixel.

3. The image processing apparatus according to claim 2,
wherein the composition unit sets the weight of the corresponding pixel to 0.

4. The image processing apparatus according to any one of claims 1 to 3,
wherein the composition unit sets the weight of the specific structure in each of the plurality of tomographic images to 1.

5. The image processing apparatus according to claim 4,
wherein the composition unit normalizes a pixel value of the specific structure on the composite two-dimensional image based on a maximum value of a pixel value that is allowed for the composite two-dimensional image.

6. The image processing apparatus according to any one of claims 1 to 5,
wherein the composition unit generates the composite two-dimensional image by applying the weight to a pixel value of a corresponding pixel of the plurality of tomographic images and adding the weighted pixel value.

7. The image processing apparatus according to any one of claims 1 to 6, further comprising:
an image acquisition unit that acquires a plurality of projection images corresponding to each of a plurality of radiation source positions, the plurality of projection images being generated by causing an imaging apparatus to perform tomosynthesis imaging of relatively moving a radiation source with respect to a detection surface of a detection unit and irradiating the subject with radiation at the plurality of radiation source positions according to movement of the radiation source; and
a reconfiguration unit that generates the plurality of tomographic images by reconfiguring the plurality of projection images.

8. The image processing apparatus according to claim 7, further comprising:
a position deviation correction unit that corrects a position deviation between the plurality of projection images,
wherein the reconfiguration unit generates the plurality of tomographic images by reconfiguring the plurality of projection images obtained by correcting the position deviation.

9. The image processing apparatus according to any one of claims 1 to 8,
wherein the composition unit performs smoothing processing on a boundary of the specific structure in the composite two-dimensional image.

10. The image processing apparatus according to any one of claims 1 to 9,
wherein the composition unit generates the composite two-dimensional image by, for pixels other than the pixel of the specific structure in each of the plurality of tomographic images and a corresponding pixel corresponding to the pixel of the specific structure included in other tomographic images, lowering a weight of a noise pixel which is more influenced by a noise than the pixel of the specific structure, or by excluding the noise pixel.

11. The image processing apparatus according to any one of claims 1 to 10,
wherein the subject is a breast and the specific structure is a spicula and a tumor.

12. An image processing method comprising:
extracting a specific structure from each of a plurality of tomographic images representing a plurality of tomographic planes of a subject; and
generating a composite two-dimensional image from the plurality of tomographic images by setting a weight of a pixel of the specific structure in each of the plurality of tomographic images to be larger than a weight of a pixel other than the pixel of the specific structure.

13. An image processing program causing a computer to execute:
a procedure of extracting a specific structure from each of a plurality of tomographic images representing a plurality of tomographic planes of a subject; and
a procedure of generating a composite two-dimensional image from the plurality of tomographic images by setting a weight of a pixel of the specific structure in each of the plurality of tomographic images to be larger than a weight of a pixel other than the pixel of the specific structure.
